# EUROPÄISCHE PATENTSCHRIFT

(11) **EP 0 688 751 B1**
(45) Veröffentlichungstag und Bekanntmachung des Hinweises auf die Patenterteilung: **07.10.1998**
(21) Anmeldenummer: 95108834.3
(22) Anmeldetag: 08.06.1995
(51) Int. Cl.: C07C 17/354, C07C 19/08

(54) **Herstellung von 1,1,1,4,4,4-Hexafluorbutan durch Umsetzung von 1,1,1,4,4,4-Hexafluorbuten mit Wasserstoff in Gegenwort eines Edelmetallkatalysators**
Preparation of 1,1,1,4,4,4-hexafluorbutane by reaction of 1,1,1,4,4,4-hexafluorbutene with hydrogen in presence of a noble metal catalyst
Préparation de butane-1,1,1,4,4,4-héxafluoré par réaction de butène-1,1,1,4,4,4 hexafluoré avec l'hydrogène en présence d'un catalyseur en métal précieux

(30) Priorität: 21.06.1994 DE 4421702
(43) Veröffentlichungstag der Anmeldung: 27.12.1995
(73) Patentinhaber: BAYER AG, 51368 Leverkusen (DE)
(72) Erfinder: Bielefeldt, Dietmar, Dr., D-40883 Ratingen (DE); Lui, Norbert, Dr., D-51061 Köln (DE); Marhold, Albrecht, Dr., D-51373 Leverkusen (DE)

(56) Entgegenhaltungen:
- EP-A- 0 442 087
- EP-A- 0 587 896
- DATABASE WPI Week 9329 Derwent Publications Ltd., London, GB; AN 93-232277 & JP-A-05 155 788 (DAIKIN KOGYO KK)

## Beschreibung

Die vorliegende Erfindung betrifft ein besonders selektives Verfahren zur Herstellung von 1,1,1,4,4,4-Hexafluorbutan (R 356) aus 1,1,1,4,4,4-Hexafluorbuten durch Hydrierung.

Es ist bereits bekannt R 356 durch Sumpfphasenhydrierung von Chlor enthaltenden Hexafluorbutenen oder chlorfreiem Hexafluorbutin herzustellen (siehe Y.Huang et al., Youji Huaxve 2 125 (1984) und DE-A 3 735 467, die der US-A 4 902 839 entspricht). Aus Chlor enthaltenden Hexafluorbutenen entsteht dabei Chlorwasserstoff, der mit Hilfe einer Base als Salz entfernt werden muß. Die Abtrennung des Salzes und dessen weiterer Verbleib erfordert besonderen Aufwand. Außerdem enthält das Reaktionsprodukt immer noch einen erheblichen Anteil Chlor enthaltender Produkte.

Nachteilig beim Einsatz von Hexafluorbutin ist dessen schwierige und kostspielige Zugänglichkeit.

Weiterhin ist ein Sumpfphasenverfahren bekannt, bei dem ebenfalls ausgehend von Hexafluorbutin R 356 erhalten wird (J. A. C. S. 71 298 (1949)). Neben der schwierigen Zugänglichkeit von Hexafluorbutin ist nachteilig, daß dabei R 356 nur in Ausbeuten von maximal 27 % der Theorie erhalten wird.

Es besteht also noch das Bedürfnis nach einem Verfahren mit dem R 356 aus gut zugänglichen Ausgangsmaterialien, in guten Ausbeuten und ohne Entsorgung von Nebenprodukten erhalten werden kann.

Es wurde nun ein Verfahren zur Herstellung von 1,1,1,4,4,4-Hexafluorbutan (R 356) gefunden, das dadurch gekennzeichnet ist, daß man in flüssiger Phase 1,1,1,4,4,4-Hexafluorbuten mit Wasserstoff in Gegenwart eines Edelmetallkatalysators umsetzt.

1,1,1,4,4,4-Hexafluorbuten ist aus 1,1,1,4,4-Pentafluor-3,4-dichlorbutan durch gleichzeitige Fluorierung und Eliminierung gut zugänglich.

Man führt das erfindungsgemäße Verfahren bei solchen Kombinationen von Druck und Temperatur durch, daß das Reaktionsgemisch (mit Ausnahme des Katalysators) im wesentlichen in flüssiger Phase vorliegt. Innerhalb dieser Vorgabe kann der Druck beispielsweise im Bereich von 0,5 bis 300 bar und die Temperatur beispielsweise im Bereich von 0 bis 250°C variiert werden. Da 1,1,1,4,4,4-Hexafluorbuten bei Normaldruck bei 9°C und 1,1,1,4,4,4-Hexafluorbutan bei Normaldruck bei 25°C siedet, wird häufig bei erhöhtem Druck gearbeitet. Bevorzugt sind Temperaturen im Bereich von 20 bis 120°C und Drucke im Bereich von 2 bis 100 bar.

Man kann das erfindungsgemäße Verfahren gegebenenfalls in Gegenwart von Lösungsmitteln durchführen. Beispielsweise kommen hier Alkohole, Ether und Kohlenwasserstoffe in Frage. Vorzugsweise setzt man aber keine Lösungsmittel zu.

Das Molverhältnis von Wasserstoff zu eingesetztem 1,1,1,4,4,4-Hexafluorbuten kann beispielsweise 100 bis 1:1 betragen. Vorzugsweise liegt es im Bereich von 10 bis 2 :1.

Bei dem Edelmetallkatalysator kann es sich z.B. um Edelmetalle und/oder Edelmetallverbindungen der VII. und/oder VIII. Nebengruppe des periodischen Systems der Elemente handeln. Diese können gegebenenfalls auf einem Träger angeordnet sein, beispielsweise auf Kieselsäure, Aluminiumoxid, Spinellen, Silikaten oder Kohlen. Als Edelmetallverbindungen kommen insbesondere Oxide, Hydroxide und Oxidhydrate in Frage. Bevorzugt sind Edelmetalle und Edelmetallverbindungen der VIII. Nebengruppe des Periodensystems der Elemente, insbesondere metallisches Palladium auf einem der genannten Trägermaterialien.

Bezogen auf 1,1,1,4,4,4-Hexafluorbuten kann man beispielsweise 0,001 bis 5 Gew.-% Katalysator (gerechnet als Metall) einsetzen. Vorzugsweise beträgt diese Menge 0,05 bis 1 Gew.-%.

Das erfindungsgemäße Verfahren kann kontinuierlich oder diskontinuierlich durchgeführt werden. Diskontinuierlich kann z.B. in einem Druckgefäß gearbeitet werden. Nach Beendigung der Reaktion kann man dabei durch Entspannen überschüssigen Wasserstoff von sonstigen Bestandteilen des Reaktionsgemisches abtrennen und gegebenenfalls wieder verwenden. Den Katalysator kann man z.B. durch Filtration (gegebenenfalls unter Druck) oder durch Abdampfung der verdampfbaren Anteile des Reaktionsgemisches (= praktisch reines 1,1,1,4,4,4-Hexafluorbutan) abtrennen. Man erhält so im allgemeinen 1,1,1,4,4,4-Hexafluorbutan in Reinheiten und Ausbeuten von über 95 %, häufig in Reinheiten über 99 % und nahezu quantitativen Ausbeuten.

Bei kontinuierlicher Arbeitsweise kann man z.B. in einem oder mehreren, hintereinandergeschalteten Reaktoren arbeiten, beispielsweise einem Kaskaden-Reaktor, die den Katalysator enthalten und denen 1,1,1,4,4,4-Hexafluorbuten und Wasserstoff zugeführt und verdampfbare Anteile des Reaktionsgemisches entnommen werden. Der Katalysator kann in den Reaktoren verbleiben, der überschüssige Wasserstoff kann durch Entspannung abgetrennt und im Kreis geführt werden. Es wird dann 1,1,1,4,4,4-Hexafluorbutan in Reinheiten und Ausbeuten wie bei der diskontinuierlichen Fahrweise erhalten.

Als Material für die Reaktionsgefäße kommt beispielsweise Edelstahl in Frage. Es ist im allgemeinen vorteilhaft, Reaktionsgefäße aus Edelstahl vor der Inbetriebnahme zu konditionieren, beispielsweise durch eine Behandlung mit Salpetersäure.

Man kann die Reaktionswärme in beiden Fällen (konti- und diskonti-Arbeitsweise) dazu benutzen, um das Produkt vom Katalysator abzudampfen. Eine weitere Reinigung des so hergestellten und isolierten 1,1,1,4,4,4-Hexafluorbutans ist im allgemeinen nicht erforderlich.

Das erfindungsgemäße Verfahren hat eine Reihe von Vorteilen: Es entstehen praktisch keine zu entsorgenden Nebenprodukte wie Chlorwasserstoff oder Salze, es geht von gut zugänglichem Ausgangsmaterial aus; es liefert 1,1,1,4,4,4-Hexafluorbutan (R 356) mit ausgezeichneten Ausbeuten und Selektivitäten, das Reaktionsgemisch kann auf einfache Weise aufgearbeitet werden, da praktisch nur überschüssiger Wasserstoff und der Katalysator vom Produkt abzutrennen sind, und es benötigt keine Zusatzstoffe wie Basen.

### Beispiele

### Beispiel 1

In einem zuvor mit 10 %iger Salpetersäure konditionierten 20-l-Edelstahlautoklaven, der mit einer Umlaufheizung und einem Ankerrührer ausgerüstet war, wurden 10,0 kg 1,1,1,4,4,4-Hexafluorbuten in Gegewart von 250 g Katalysator (5 Gew.-% metallisches Palladium auf Aktivkohle) bei einem Druck von 20 bar Wasserstoff innerhalb von 10 Minuten von 0 auf 30°C erwärmt. Nach dem Erreichen dieser Temperatur wurde der Wasserstoffverbrauch ausgeglichen durch mehrmaliges Aufdrücken von frischem Wasserstoff bei Drucken zwischen 20 und 30 bar. Nach ca. 2,5 Stunden lag der Umsatz bei 87 %. 1,1,1,4,4,4-Hexafluorbutan hatte sich in einer Ausbeute von größer als 99 % der Theorie gebildet.

### Beispiel 2

Es wurde verfahren wie in Beispiel 1, jedoch wurde die Hydrierung bei 50°C durchgeführt und nach Beendigung der Wasserstoffaufnahme noch 3 Stunden bei 50°C nachgerührt. Danach wurde 1,1,1,4,4,4-Hexafluorbutan durch Druckfiltration vom Katalysator abgetrennt. Bei einem Umsatz von über 99,8 % wurde 1,1,1,4,4,4-Hexafluorbutan praktisch quantitativ erhalten.

### Beispiel 3

Es wurde verfahren wie in Beispiel 1, jedoch wurde die Hydrierung bei 70°C und bei Drucken von 40 bis 60 bar Wasserstoff durchgeführt. Nach Beendigung der Wasserstoffaufnahme wurde noch 1 Stunde bei 70°C nachgerührt. Danach wurde 1,1,1,4,4,4-Hexafluorbutan durch Druckfiltrafion vom Katalysator abgetrennt. Das 1,1,1,4,4,4-Hexafluorbuten hatte sich praktisch quantitativ umgesetzt.

## Patentansprüche

1. Verfahren zur selektiven Herstellung von 1,1,1,4,4,4-Hexafluorbutan (R 356), dadurch gekennzeichnet, daß man in flüssiger Phase 1,1,1,4,4,4-Hexafluorbuten mit Wasserstoff bei Drucken im Bereich von 0,5 bis 100 bar und Temperaturen im Bereich von 0 bis 250° in Gegenwart eines Edelmetallkatalysators aus Edelmetallen und/oder Edelmetallverbindungen der VII. und/oder VIII. Nebengnlppe des Periodensystems der Elemente umsetzt.

2. Verfahren nach Anspruch 1, dadurch gekennzeichnet, daß man es bei Drucken im Bereich von 2 bis 100 bar und Temperaturen im Bereich von 20 bis 120°C durchführt.

3. Verfahren nach Ansprüchen 1 bis 2, dadurch gekennzeichnet, daß das Molverhaltnis von Wasserstoff zu eingesetztem 1,1,1,4,4,4-Hexafluorbuten 100 bis 1:1 beträgt.

4. Verfahren nach Ansprüchen 1 bis 3, dadurch gekennzeichnet, daß es sich bei dem Edelmetallkatalysator um metallisches Palladium handelt.

5. Verfahren nach Ansprüchen 1 bis 4, dadurch gekennzeichnet, daß der Edelmetallkatalysator auf einem Träger angeordnet ist.

6. Verfahren nach Ansprüchen 1 bis 5, dadurch gekennzeichnet, daß man 0,001 bis 5 Gew.-% Edelmetallkatalysator (gerechnet als Metall) einsetzt.

7. Verfahren nach Ansprüchen 1 bis 6, dadurch gekennzeichnet, daß man es kontinuierlich durchführt.

8. Verfahren nach Ansprüchen 1 bis 6, dadurch gekennzeichnet, daß man es diskontinuierlich durchführt.

## Claims

1. Process for selectively preparing 1,1,1,4,4,4-hexafluorobutane (R 356), characterized in that 1,1,1,4,4,4-hexafluorobutene is reacted in the liquid phase with hydrogen at pressures in the range from 0.5 to 100 bar and at temperatures in the range from 0 to 250° in the presence of a noble metal catalyst comprising noble metals and/or noble metal compounds of transition group VII and/or VIII of the Periodic Table of the Elements.

2. Process according to Claim 1, characterized in that it is carried out at pressures in the range from 2 to 100 bar and at temperatures in the range from 20 to 120°C.

3. Process according to either Claim 1 or 2, characterized in that the molar ratio of hydrogen to 1,1,1,4,4,4-hexafluorobutene used is from 100 to 1:1.

4. Process according to any of Claims 1 to 3, characterized in that the noble metal catalyst is metallic palladium.

5. Process according to any of Claims 1 to 4, characterized in that the noble metal catalyst is arranged on a support.

6. Process according to any of Claims 1 to 5, characterized in that from 0.001 to 5% by weight of noble metal catalyst (calculated as metal) is used.

7. Process according to any of Claims 1 to 6, characterized in that it is carried out continuously.

8. Process according to any of Claims 1 to 6, characterized in that it is carried out batchwise.

## Revendications

1. Procédé pour la préparation sélective du 1,1,1,4,4,4-hexafluorobutane (R 356), caractérisé en ce qu'on fait réagir en phase liquide du 1,1,1,4,4,4-hexafluorobutène avec de l'hydrogène sous des pressions dans le domaine de 0,5 à 100 bar et à des températures dans le domaine de 0 à 250°C en présence d'un catalyseur de métal noble constitué par des métaux nobles et/ou par des composés de métaux nobles des sous-groupes VII et/ou VIII du tableau périodique des éléments.

2. Procédé selon la revendication 1, caractérisé en ce qu'on l'effectue sous des pressions dans le domaine de 2 à 100 bar et à des températures dans le domaine de 20 à 120°C.

3. Procédé selon les revendications 1 à 2, caractérisé en ce que le rapport molaire de l'hydrogène au 1,1,1,4,4,4-hexafluorobutène mis en oeuvre s'élève de 100 à 1:1.

4. Procédé selon les revendications 1 à 3, caractérisé en ce qu'il s'agit, en ce qui concerne le catalyseur de métal noble, de palladium métallique.

5. Procédé selon les revendications 1 à 4, caractérisé en ce que le catalyseur de métal noble est déposé sur un support.

6. Procédé selon les revendications 1 à 5, caractérisé en ce qu'on met en oeuvre le catalyseur de métal noble (calculé sous forme de métal) à concurrence de 0,001 à 5% en poids.

7. Procédé selon les revendications 1 à 6, caractérisé en ce qu'on l'effectue en continu.

8. Procédé selon les revendications 1 à 6, caractérisé en ce qu'on l'effectue en discontinu.
